# EUROPEAN PATENT SPECIFICATION

(11) **EP 3 904 408 B1**
(45) Date of publication and mention of the grant of the patent: **20.11.2024**
(21) Application number: 19903132.9
(22) Date of filing: 26.12.2019
(51) Int. Cl.: C08F 220/28, C08F 220/30, A61F 2/16, A61L 27/16

(54) **SOFT INTRAOCULAR LENS MATERIAL AND SOFT INTRAOCULAR LENS**
WEICHES INTRAOKULARLINSENMATERIAL UND WEICHE INTRAOKULARLINSE
MATÉRIAU POUR LENTILLE INTRAOCULAIRE SOUPLE ET LENTILLE INTRAOCULAIRE SOUPLE

(30) Priority: 28.12.2018 WO PCT/JP2018/048610
(43) Date of publication of application: 03.11.2021
(73) Proprietor: Nidek Co., Ltd., Gamagori-shi, Aichi, 443-0038 (JP)
(72) Inventor: TOYODA, Takahiro, Gamagori-shi, Aichi 443-0038 (JP); MURASE, Kyoko, Gamagori-shi, Aichi 443-0038 (JP)
(74) Representative: Kuhnen & Wacker Patent- und Rechtsanwaltsbüro PartG mbB
(86) International application number: PCT/JP2019/051262
(87) International publication number: WO 2020/138346

(56) References cited:
- WO-A1-2006/095750
- WO-A1-2018/021455
- WO-A2-2012/004744
- JP-A- H01 280 464
- JP-A- H07 157 523
- JP-A- H08 173 522
- US-B1- 10 117 965

## Description

### BACKGROUND OF THE INVENTION

### Technical Field

The present invention relates to a soft intraocular lens material and a soft intraocular lens.

The present application claims priority from International Application No. PCT/JP2018/048610 filed on December 28, 2018.

### Background Art

Conventionally, as a method of treatment for cataract, a method in which a clouded crystalline lens is removed from a crystalline lens capsule and an intraocular lens (IOL) exerting a refractive force, instead of the crystalline lens is inserted into the capsule has been widely employed. An intraocular lens includes an optical part expressing a refractive force and a support part fixing a position of the optical part in the capsule. In inserting the intraocular lens, for example, one piece-type intraocular lens constituted by integrally forming the optical part and the support part is prepared and the intraocular lens is inserted into an eye in a folded state using an insertion tool called an injector. The inserted intraocular lens is restored to an original shape in the capsule and, with the support part pressed against the capsule, a position of the intraocular lens in the capsule is stabilized. The intraocular lens is desired to have high flexibility and shape recovery property in order to make an incision in the crystalline lens capsule as small as possible (see, for example, Patent Documents 1 to 3). Moreover, the Patent Documents 4 and 5 disclose intraocular lens material according to prior art.

### CITATION LIST

### PATENT DOCUMENT

PATENT DOCUMENT 1: WO 2018/021455 A1
PATENT DOCUMENT 2: JP H11-56998 A
PATENT DOCUMENT 3: WO 2016/121804 A1
PATENT DOCUMENT 4: WO 2012/004744 A2
PATENTDOCUMENT 5: US 10117965 B1

### SUMMARY OF THE INVENTION

### Problem to be Solved by Invention

As a constituent material of such a foldable soft intraocular lens, a soft resin made of silicone, hydrophobic (meth)acrylate, hydrophilic (meth)acrylate, or the like has been generally used. Among those compounds, hydrophobic (meth)acrylate is widely employed because there are fewer cases of secondary cataract after surgery. Hydrophobic (meth)acrylate has low affinity with moisture and a phenomenon called glistening that a very little amount of moisture in the lens is condensed in a dot form and looks shining tends to occur. Therefore, it is indispensable in producing soft intraocular lens materials to use hydrophobic (meth acrylate and hydrophilic (meth)acrylate in combination. However, for known soft intraocular lens materials, there is room for improvement in achieving both flexibility and shape recovery property that have been described above and reduction of glistening. Diversity of soft intraocular lens materials that can achieve both the flexibility and the shape recovery property that have been described above and reduction of glistening is potentially required.

In view of the present circumstances of the above-described conventional technology, it is an object of the present application to provide a new soft intraocular lens material that can achieve both the flexibility and the shape recovery property and the reduction of glistening. Moreover, from another viewpoint, it is another object of the present application to provide a soft intraocular lens using the soft intraocular lens material.

### Solution to Problem

A soft intraocular lens material that is provided by the technology disclosed herein is obtained by polymerizing polymerizable polymer components, the polymer components include an aromatic ring-containing methacrylate (A), an alkoxy group-containing acrylate (B), a hydroxy group-containing acrylate (C), and a cross-linkable (meth)acrylate (D), given that a total content of the polymer components is 100 parts mass, a ratio of the aromatic ring-containing methacrylate (A) is 40 parts mass or more and 52 parts mass or less. Given that a total content of the polymer components is 100 parts mass, a ratio of the alkoxy group-containing acrylate (B) is 35 parts mass or more and 46 parts mass or less. Given that a total content of the polymer components is 100 parts mass, a ratio of the hydroxy group-containing acrylate (C) is 8 parts mass or more and 12 parts mass or less, given that a total content of the polymer components is 100 parts mass, a ratio of the cross-linkable (meth)acrylate (D) is 1 part mass or more and 10 parts mass or less, wherein the aromatic ring-containing methacrylate (A) contains a methacrylate represented by a general formula (1): 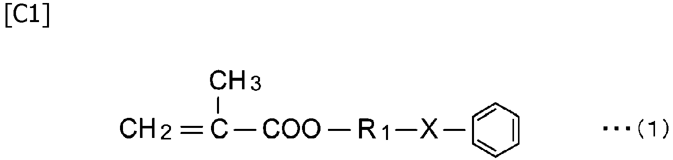
where R₁ is a linear or branched alkylene group having 1 to 8 carbon atoms and X indicates that an element is absent or is an oxygen atom,
   the alkoxy group-containing acrylate (B) contains an acrylate represented by a general formula (2): 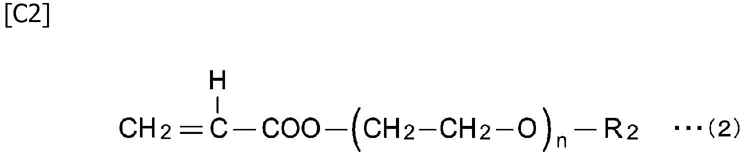
where R₂ is a methyl group or an ethyl group and n is an integer of 1 to 4,
   the hydroxy group-containing acrylate (C) contains an acrylate represented by a general formula (3): 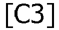 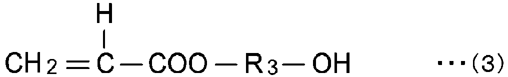
where R₃ is a linear or branched alkylene group having 1 to 8 carbon atoms, and the cross-linkable (meth)acrylate (D) is a bifunctional (meth)acrylate or a trifunctional (meth)acrylate.

As a result of detailed examination of a known soft intraocular lens material conducted by the inventors of the present invention, the following matters were found. That is, the known soft intraocular lens material sufficiently contains a hydrophilic (meth)acrylate to suppress the occurrence of glistening. However, although the hydrophilic (meth)acrylate can suppress the occurrence of glistening by creating an environment where aqueous humor is less likely to be flocculate in a cavity of a polymer, hardness of the polymer is increased contrarily. Moreover, when hydrophobicity of the soft intraocular lens material is increased, the soft intraocular lens material absorbs moisture in air and a shape or physical properties of a lens are easily changed, so that it becomes difficult to handle the intraocular lens. Note that, among hydrophobic (meth)acrylates, monomer components, such as a known polymethyl methacrylate (PMMA) or the like, that can suppress the occurrence of glistening can exist, but a polymer obtained using such monomer components is a hard material that is so hard that a lens formed of the polymer cannot be folded, and thus, is difficult to use.

Therefore, through an intensive study, the inventors of the present invention found a following knowledge and reached the completion of the present invention. The glistening suppression effects are expressed without impairing the flexibility and the shape recovery property (which will be hereinafter occasionally referred to as "physical characteristics") suitable for the soft intraocular lens material, by combining a methacrylate monomer containing an aromatic ring and an alkoxy acrylate that does not contain an aromatic ring. That is, the function of the mixture of the methacrylate monomer containing an aromatic ring and the alkoxy acrylate that does not contain an aromatic ring can substitute a part of a function of a hydrophilic monomer. However, in a point of ensuring sufficient flexibility, it is important that 30 parts mass or more of the alkoxy group-containing acrylate (B) that is softer than a methacrylate component is contained. Accordingly, a soft intraocular lens material in which both the flexibility and the shape recovery property and the reduction of glistening have been achieved is provided.

According to the soft intraocular lens material of the present invention, given that a total content of the polymer components is 100 parts mass, a ratio of the aromatic ring-containing methacrylate (A) is 40 parts mass or more and 52 parts mass or less. According to this structure, the occurrence of glistening can be preferably suppressed without impairing the flexibility suitable for folding of the soft intraocular lens material.

According to the soft intraocular lens material of the present invention, given that a total content of the polymer components is 100 parts mass, a ratio of the alkoxy group-containing acrylate (B) is 35 parts mass or more and 46 parts mas or less. According to this structure, both the flexibility and the shape recovery property and the reduction of glistening can be preferably achieved.

According to the soft intraocular lens material of the present invention, given that a total content of the polymer components is 100 parts mass, a ratio of the hydroxy group-containing acrylate (C) is 8 parts mass or more and 12 parts mass or less. By suppressing use of a hydrophilic acrylate to such a small content, reduction in the flexibility and the shape recovery property in a soft intraocular lens can be further reduced.

In one preferred embodiment of the soft intraocular lens material disclosed herein, given that a total content of the polymer components is 100 parts mass, a total among of the aromatic ring-containing methacrylate (A) and the alkoxy group-containing acrylate (B) is 75 parts mass or more and 90 parts mass or less. According to the soft intraocular lens material disclosed herein, it is considered that a polymer having an effective skeleton for reduction of glistening is formed by a combination of the aromatic ring-containing methacrylate and the alkoxy group-containing acrylate. As a result, in a state where a content of a hard hydrophilic (meth)acrylate is reduced, glistening can be reduced, and the physical characteristics and reduction of glistening can be balanced at a high level.

According to the soft intraocular lens material of the present invention, the aromatic ring-containing methacrylate (A) contains a methacrylate represented by a general formula (1): 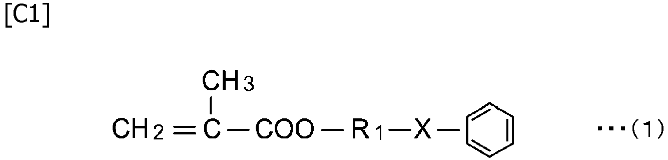 where R₁ is a linear or branched alkylene group having 1 to 8 carbon atoms and X indicates that an element is absent or is an oxygen atom. Thus, the above-described effects can be further increased while a high refractive index and high flexibility of the soft intraocular lens material are realized.

According to the soft intraocular lens material of the present invention, the alkoxy group-containing acrylate (B) contains an acrylate represented by a general formula (2): 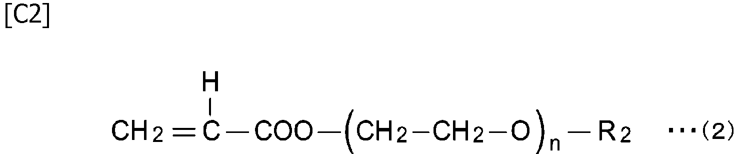 where R₂ is a methyl group or an ethyl group and n is an integer of 1 to 4. Thus, the above-described effects can be further increased.

According to the soft intraocular lens material of the present invention, the hydroxy group-containing acrylate (C) contains an acrylate represented by a general formula (3): 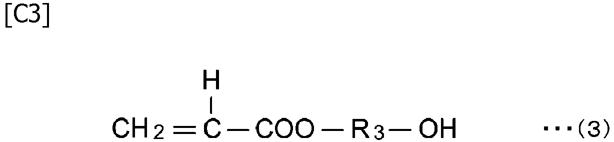 where R₃ is a linear or branched alkylene group having 1 to 8 carbon atoms. Thus, the above-described effects can be further increased.

In one preferred embodiment of the soft intraocular lens material disclosed herein, the polymer components include a monomer having an ultraviolet absorbing ability. Thus, a soft intraocular lens that can reduce a risk of developing an eye disease resulting from ultraviolet radiation is provided.

In one preferred embodiment of the soft intraocular lens material disclosed herein, the polymer components include a monomer having a yellow coloring ability and a copolymerizability with the (meth)acylate monomer component, wherein the monomer is an azo-based compound or a pyrazole-based compound. Thus, transmission of blue light can be partially suppressed and a natural ocular vision like an ocular vision through a crystalline lens without strange feeling can be provided.

The above-described soft intraocular lens materials have excellent flexibility and glistening suppression effects. These characteristics are particularly preferable when the soft intraocular lens material is used as a material forming a soft intraocular lens that is kept in a state where the soft intraocular lens is implanted inside an eyeball and then is immersed in aqueous humor for a long time. Therefore, in another aspect, the technology disclosed herein provides a soft intraocular lens produced using the above-described soft intraocular lens material described in the above-described embodiments.

### BRIEF DESCRIPTION OF THE DRAWINGS

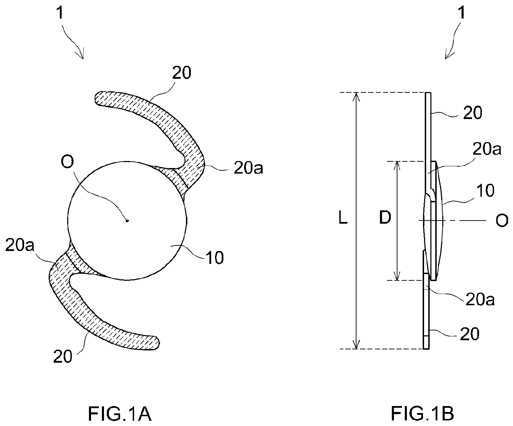

[FIG. 1] FIG. 1(a) and FIG. 1(b) are a plan view and a side view schematically illustrating a structure of a one-piece type intraocular lens according to an embodiment.

### DETAILED DESCRIPTION OF THE PREFERRED EMBODIMENTS

Preferred embodiments of the present invention will be described below. Note that a person skilled in the art can understand matters other than matters specifically mentioned in this specification (composition of a soft intraocular lens or the like) and necessary for the implementation of the present invention (basic form of the intraocular lens or the like), based on teaching for carrying out the present invention described in this specification and the common general technical knowledge at the time of filing. The present invention can be carried out based on the contents disclosed in this specification and the common general technical knowledge in the field. Moreover, in the following drawings, members/parts that have the same effect may be denoted by the same sign and the overlapping description may be omitted or simplified. The notation "A to B" indicating a numerical range means "A or more and B or less" unless otherwise noted.

FIG. 1(a) and FIG. 1(b) are views illustrating a structure of a one-piece type intraocular lens according to an embodiment. FIG. 1(a) is a plan view and FIG. 1(b) is a side view. A soft intraocular lens 1 includes an optical part 10 having a predetermined refractive force and a pair of support parts 20 supporting the optical part 10 in an eye. The optical part 10 and the support parts 20 are integrally formed of the same raw material resin composition.

The optical part 10 has a circular shape with a diameter D of approximately 5.5 mm to 7 mm, typically, approximately 6 mm, when viewed from top. A direction perpendicular to a direction of the diameter D of the optical part 10 is a thickness direction. The optical part 10 has, for example, a biconvex lens shape that protrudes toward both sides (both side in the thickness direction), when viewed from side. A thickness of the optical part 10 can be determined based on a refractive index of a material forming the optical part 10, a desired refractive force required for the optical part 10, or the like. At a center O of the circular optical part 10, the thickness of the optical part 10 is, for example, 300 µm or more, and may be 400 µm or more as one example. The thickness of the optical part 10 is, for example, approximately 1000 µm or less, that is, for example, 900 µm or less, and may be 700 µm or less, that is, approximately 600 µm (approximately plus or minus 10%) as an example. However, the shape of the optical part 10 is not limited thereto. The shape of the optical part 10 may be, for example, an oval shape or a nearly oval shape when viewed from top. The shape of the optical part 10 may be, for example, a planoconvex lens shape in which one surface protrudes and the other surface is flat, or may be a convex meniscus lens shape in which one surface protrudes and the other surface is concave, or the like.

The support part 20 is formed so as to protrude outward from a periphery of the optical part 10. The pair of optical part 10 is arranged with respect to the optical part 10 in point symmetry with respect to the center O of the optical part 10 serving as a symmetric axis. The support part 20 has a loop shape in which one end is a free end. Each of the pair of support parts 20 includes a bending part 20a largely bending in a plane surface of a lens part near a connection portion with the optical part 10. The support part 20 is configured to be further bendable in the bending part 20a in a direction in which a free end part becomes closer to the center. A distance L between the respective free ends of the pair of support parts 20 is, for example, approximately 11.5 mm to 13.5 mm, typically, approximately 12 mm to 13 mm. However, the shape of the support parts 20 is not limited thereto. The optical part 10 and the support parts 20 may be formed of the same material, and may be formed of different materials. In the soft intraocular lens 1 disclosed herein, for example, the optical part 10 and the support parts 20 may be formed of the same soft intraocular lens material.

The soft intraocular lens material is formed by polymerizing polymerizable polymer components. The polymer components forming the soft intraocular lens material essentially include an aromatic ring-containing methacrylate (A), an alkoxy group-containing acrylate (B), a hydroxy group-containing acrylate (C), and a cross-linkable (meth)acrylate (D). In other words, the soft intraocular lens material is formed of a (meth)acrylate copolymer containing a monomer unit corresponding to the aromatic ring-containing methacrylate (A), a monomer unit corresponding to the alkoxy group-containing acrylate (B), a monomer unit corresponding to the hydroxy group-containing acrylate (C), and a monomer unit corresponding to the cross-linkable (meth)acrylate (D).

Note that, as used in the present specification, the term "(meth)acrylate copolymer" refers to polymers collectively called "acrylic polymer," and the (meth)acrylate copolymer is a copolymer containing a monomer unit (which will be hereinafter occasionally referred to as a (meth)acrylate monomer) derived from a monomer having at least one (meth)acryloyl group containing a polymerizable unsaturated double bond in one molecular structure. The (meth)acrylate copolymer is typically a polymer containing a monomer unit derived from a (meth)acrylate monomer at a ratio of 50 percent by mass or more (preferably 75 percent by mass or more, more preferably 80 percent by mass or more, and particularly preferably 90 percent by mass, that is, for example, 95 percent by mass or more) in total in one molecule structure. Note that "(meth)acrylate" is a term collectively referring to acrylate and methacrylate. Each polymer component will be described below.

The inventors of the present invention used the aromatic ring-containing methacrylate (A) and the alkoxy group-containing acrylate (B) as main polymer components based on the knowage that combination use of the aromatic ring-containing methacrylate (A) and the alkoxy group-containing acrylate (B) having an effect of achieving both appropriate physical characteristics and glistening suppression at a high level. In other words, the aromatic ring-containing methacrylate (A) and the alkoxy group-containing acrylate (B) cooperate to contribute to achieving both appropriate physical characteristics and glistening suppression effect. Although details of the above-described mechanism are not clearly understood, according to studies conducted by the inventors of the present invention so far, it has been found that a methacrylate polymer component can exist near a surface of a polymer at a relatively high concentration, as compared to an acrylate polymer component (see, for example, WO 2019/138952 A1). Even among polymers generally called hydrophobic acrylic polymer, many polymers have a water absorbing property although they can contain water at less than 1 percent, and it is considered that glistening occurs based on this small water absorbing property in a generic intraocular lens. In contrast, when being polymerized at a high concentration, the aromatic ring-containing methacrylate (A) disclosed herein can exhibit high hydrophobicity similar to that of PMMA. Therefore, it is considered that, according to the structure disclosed herein, the aromatic ring-containing methacrylate (A) is polymerized near a surface at a high concentration to form a hydrophobic surface and thus effectively contributes to reduction of glistening. It is also considered that it is possible to cause the alkoxy group-containing acrylate (B) and the hydroxy group-containing acrylate (C) that can possibly have hydrophobicity to exist near a center of the polymer at a relatively high concentration, so that glistening suppression at the surface of the polymer is achieved and excellent flexibility and shape recovery property are given to the polymer.

As the polymerizable aromatic ring-containing methacrylate (A), a compound containing at least one aromatic hydrocarbon group in a structure thereof. A methacrylate monomer containing an aromatic ring gives a function of increasing the refractive index in the intraocular lens to the polymer. Examples of the aromatic hydrocarbon group include a benzene ring, a naphthalene ring, a biphenyl ring, a heterocyclic ring, or the like. Examples of the heterocyclic ring include a morpholine ring, a piperidine ring, a pyrrolidine ring, a piperazine ring, or the like. Specific examples of aromatic ring-containing methacrylic monomers include, for example, those having a benzene ring, such as a benzyl methacrylate, a phenyl methacrylate, an o-phenylphenol methacrylate, a phenoxy methacrylate, an ethylene glycol phenyl ether methacrylate, a diethylene glycol phenyl ether methacrylate, a propylene glycol phenyl ether methacrylate, an ethylene oxide modified nonylphenol methacrylate, an ethylene oxide modified cresol methacrylate, a phenol ethylene oxide modified methacrylate, a 2-hydroxy-3-phenoxypropyl methacrylate, a methoxybenzyl methacrylate, a chlorobenzyl methacrylate, a cresyl methacrylate, a polystyryl methacrylate, or the like; those having a naphthalene ring, such as a hydroxyethylated β-naphthol methacrylate, a 2-naphthoethyl methacrylate, a 2-(4-methoxy-1-naphthoxy)ethyl methacrylate, or the like; those having a biphenyl ring, such as a biphenyl methacrylate or the like, or the like. One of these aromatic ring-containing methacrylic monomers may be included alone or two or more of them may be included in combination. Given that a total of four types of polymer components, that is, the above-described polymer components (A) to (D), is, for example, 100 parts mass, a content of the polymerizable aromatic ring-containing methacrylate monomer in the (meth)acrylate copolymer is 40 parts mass or more, and may be, for example, about 42 parts mass or more, although the ratio is not limited to those described above. The ratio of the aromatic ring-containing methacrylate (A) is 52 parts mass or less, and may be, for example, about 50 parts mass or less.

Incidentally, it is known that a high polymer containing an aromatic ring-containing methacrylate monomer as a unit tends to have high hardness because of a structure having a large aromatic ring. Therefore, an aromatic ring-containing (meth)acrylic monomer is preferably a methacrylate monomer having a relatively low glass transition point. The aromatic ring-containing methacrylate (A) described above is a methacrylate monomer represented by the following general formula (1): 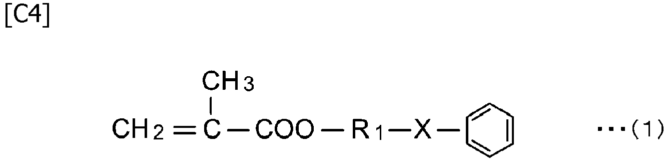 In the general formula (1), R₁ is a linear or branched alkylene group having 1 to 8 carbon atoms and X indicates that an element is absent (that is, a single bond) or is an oxygen atom. The alkylene group can be specifically, for example, a methylene group, an ethylene group, an n-propylene group, an isopropylene group, a cyclopropylene group, an n-butylene group, an isobutylene group, an s-butylene group, a t-butylene group, a cyclobutylene group, an n-pentylene group, a 1-methyl-n-butylene group, a 2-methyl-n-butylene group, a 3-methyl-n-butylene group, a 1,1-dimethyl-n-propylene group, a 1,2-dimethyl-n-propylene group, a 2,2-dimethyl-n-propylene group, a 1-ethyl-n-propylene group, a cyclopentylene group, an n-hexylene group, or the like. From a viewpoint of reducing the water absorbing property of a polymer, X is more preferably a single bond. Preferred examples of such methacrylate monomer components include a benzyl methacrylate, a phenylethyl methacrylate, an ethylene glycol monophenyl ether methacrylate, a diethylene glycol monophenyl ether methacrylate, a tetraethylene glycol monophenyl ether methacrylate, or the like.

The alkoxy group-containing acrylate (B) is, for example, an acrylate monomer component represented by the formula: CH₂ = CHCOOR₂, and R₂ is a monomer that is a functional group and/or a substituent containing an alkoxy group at least in a part thereof. The alkoxy group may be, for example, an alkoxy group having 1 to 5 carbon atoms (which will be hereinafter referred to merely as "C₁₋₅"), C₁₋₄, typically, C₁, C₂, C₃, or the like. Specific examples of the alkoxy group-containing acrylate (B) include a methoxy group, an ethoxy group, a propoxy group, a t-butoxy group, a pentyloxy group, an allyloxy group, or the like, and functional groups partially containing these.

Specific examples of such alkoxy group-containing acrylates include, for example, alkoxy group-containing acrylates, such as an ethylene glycol monomethyl ether acrylate, an ethylene glycol monoethyl ether acrylate, an ethylene glycol monobutyl acrylate, a diethylene glycol monomethyl ether acrylate, a diethylene glycol monoethyl ether acrylate, a diethylene glycol monobutyl ether acrylate, a dipropylene glycol methyl ether acrylate, a dipropylene glycol ethyl ether acrylate, a triethylene glycol monomethyl ether acrylate, a triethylene glycol monoethyl ether acrylate, a tetraethylene glycol monomethyl ether acrylate, a tetraethylene glycol monoethyl ether acrylate, a polyethylene glycol monomethyl ether acrylate, a polyethylene glycol monoethyl ether acrylate, a 2-(2-ethylhexaoxy)ethyl acrylate, or the like. The alkoxy group-containing acrylate (B) contains an acrylate represented by the following general formula (2). 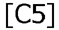 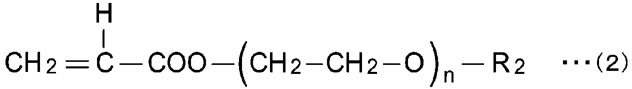 In the general formular (2), R₂ is a methyl group or an ethyl group and n is an integer of 1 to 4. Examples of such compounds include an ethoxy group monomethyl ether acrylate containing 1 to 4 ethoxy groups, ethoxy group monoethyl ether acrylate containing 1 to 4 ethoxy groups, or the like. Among these compounds, use of an ethylene glycol monomethyl ether acrylate (2-methoxyethyl acrylate: MEA), an ethylene glycol monoethyl ether acrylate (2-ethoxyethyl acrylate: EEA), a diethylene glycol monomethyl ether acrylate (2-(2-ethoxyethoxy) methyl acrylate: EEMA), or a diethylene glycol monoethyl ether acrylate (2-(2-ethoxyethoxy)emethyl acrylate: EEEA) is preferable. One of the above-described compounds may be included alone and two or more of the above-described may be included in combination. Given that a total of four types of polymer components, that is, the above-described polymer components (A) to (D), is, for example, 100 parts mass, a ratio of the alkoxy group-containing acrylate (B) in the polymer components is 35 parts mass or more, and may be, for example, about 38 parts mass or more. The ratio of the alkoxy group-containing acrylate (B) is 46 parts mass or less, and may be, for example, about 40 parts mass or less.

The hydroxy group-containing acrylate (C) has a function of increasing hydrophilicity of the (meth)acrylate copolymer, creating an environment where aqueous humor is less likely to be flocculate in a cavity of the copolymer, and suppressing the occurrence of glistening. The hydroxy group-containing acrylate (C) contains a compound represented by the following general formular (3): 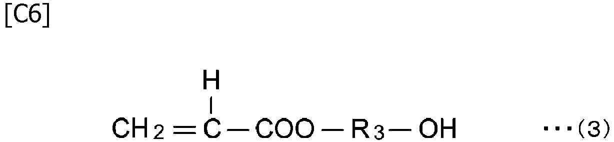 In the general formula (3), R₃ is a linear or branched alkylene group having 1 to 8 carbon atoms. Examples of such polymer components include a 2-hydroxyethyl acrylate, a 2-hydroxypropyl acrylate, a 4-hydroxybutyl acrylate, a 6-hydroxyhexyl acrylate, a 2,3-dihydroxypropyl acrylate, and an 8-hydroxyoctyl acrylate, or the like. Among these compounds, use of the 2-hydroxyethyl acrylate (HEA), the 4-hydroxybutyl acrylate (HBA), or the like is preferable. One of the above-described compounds may be included alone and two or more of the above-described may be included in combination.

A ratio of the hydroxy group-containing acrylate (C) in the (meth)acrylate copolymer is not strictly limited. For example, from a viewpoint of preferably suppressing glistening of the intraocular lens, given that a total of four types of polymer components, that is, the above-described polymer components (A) to (D), is, for example, 100 parts mass, the ratio of the hydroxy group-containing acrylate (C) is 8 parts mass or more, and may be, for example, about 9 parts mass or more. However, in the technology disclosed herein, because the aromatic ring-containing methacrylate (A) and the alkoxy group-containing acrylate (B) suppress the occurrence of glistening under more relaxed conditions, the ratio of the hydroxy group-containing acrylate (C) that can possibly affect a shape, physical properties, such as a water content, or the like is preferably small. From the above-described viewpoint, the ratio of the hydroxy group-containing acrylate (C) is 12 parts mass or less or 11.5 parts mass or less, and may be, for example, about 11 parts mass or less.

The cross-linkable (meth)acrylate (D) is a bifunctional (meth)acrylate or a polyfunctional (meth)acrylate having three functionalities. In a general polymer design, to introduce a crosslinking point, a monomer having three to six functionalities is generally used. However, there is a probability that, as the functionalities increase, the hardness of a polymer increases. In a case where a crosslinking point is introduced in the soft intraocular lens material, it is required to ensure appropriate hardness and high flexibility, and therefore, a bi- or trifunctional (meth)acrylate is used. The cross-linkable (meth)acrylate (D) is configured not to include a polyfunctional (meth)acrylate having four or more functionalities.

Specific examples of such bifunctional (meth)acrylates include, for example, a 1,3-butylene glycol di(meth)acrylate, a 1,4-butandiol di(meth)acrylate, a 1,6-hexandiol di(meth)acrylate, a 1,9-nonanediol di(meth)acrylate, a 1,10-decandiol di(meth)acrylate, an ethylene glycol di(meth)acrylate, a diethylene glycol di(meth)acrylate, a triethylene glycol di(meth)acrylate, a tetramethylene glycol di(meth)acrylate, an ethylene oxide modified bisphenol A di(meth)acrylate, a propylene glycol di(meth)acrylate, a dipropylene glycol di(meth)acrylate, a tripropylene glycol di(meth)acrylate, or the like. Specific examples of the trifunctional (meth)acrylate include, for example, a trimethylolpropane tri(meth) acrylate, a tris (2-acryloyloxyethyl) isocyanurate, an ethylene oxide modified trimethylolpropane tri(meth) acrylate, or the like. Among these compounds, any one of a 1,3-butylene glycol di(meta)acrylate, a 1,4-butanediol di(meta)acrylate, and a 1,6-hexanediol di(meta)acrylate is preferably used. Note that, given that a total amount of the cross-linkable (meth)acrylate (D) is 100 percent by mass, a ratio of a cross-linkable (meth)acrylate component is preferably about 50 percent by mass or more, is more preferably about 60 percent by mass or more, is preferably, for example, about 70 percent by mass or more, about 80 percent by mass or more, or about 90 percent by mass, and may be, for example, substantially, 100 percent by mass, although the ratio of the cross-linkable (meth)acrylate component is not limited to those described above. Thus, the physical properties of the soft intraocular lens material can be stably increased.

A ratio of the cross-linkable (meth)acrylate (D) in the (meth)acrylate copolymer is not strictly limited. For example, from a viewpoint of preferably suppressing glistening of the soft intraocular lens, given that a total of four types of polymer components, that is, the above-described polymer components (A) to (D), is, for example, 100 parts mass, the ratio of the cross-linkable (meth)acrylate (D) is 1 parts mass or more, is preferably about 3 parts mass or more, and may be, for example, about 4 parts mass or more. However, when an amount of the cross-linkable (meth)acrylate (D) is excessive, there is a probability that the hardness of the soft intraocular lens material becomes too high, it is not preferable that the amount of the cross-linkable (meth)acrylate (D) is excessive. In view of this point, the ratio of the cross-linkable (meth)acrylate (D) is 10 parts mass or less, is more preferably 8 parts mass or less, and may be, for example, 5 parts mass or less.

In the aromatic ring-containing methacrylate (A), the alkoxy group-containing acrylate (B), the hydroxy group-containing acrylate (C), and the cross-linkable (meth)acrylate (D) that have been described above, given that a ratio of a total mol number M_{M} of methacryl monomers to a total mol number M_{A} of acrylic monomers is M_{M}/M_{A}, a value of M_{M}/M_{A} is preferably 0.2 or more, is more preferably 0.4 or more, and may be, for example, 0.5 or more, although the ratio is not limited to those described above. Moreover, the value of M_{M}/M_{A} is preferably 1. 2 or less, is more preferably 1.1 or less, and may be, for example, 1 or less.

From a viewpoint of enhancing an effect of a combination of the aromatic ring-containing methacrylate (A) and the alkoxy group-containing acrylate (B), given that a total of four types of polymer components, that is, the above-described polymer components (A) to (D), is, for example, 100 parts mass, a total amount of the aromatic ring-containing methacrylate (A) and the alkoxy group-containing acrylate (B) may be 75 parts mass or more, is properly 80 parts mass or more, or may be 81 parts mass or more, 82 parts mass or more, 83 parts mass or more, or 85 parts mass or more. In other words, a total amount of the hydroxy group-containing acrylate (C) and the cross-linkable (meth)acrylate (D) may be 25 parts mass or less, is properly 20 parts mass or less, or may be 19 parts mass or less, 18 parts mass or less, 17 parts mass or less, or 15 parts mass or less.

However, considering contents of a hydrophilic monomer component and a crosslinking monomer component, the total amount of the aromatic ring-containing methacrylate (A) and the alkoxy group-containing acrylate (B) is preferably set to 90 parts mass or less, for example, 89 parts mass or less, or about 88 parts mass or less. In other words, the total amount of the hydroxy group-containing acrylate (C) and the cross-linkable (meth)acrylate (D) may be 10 parts mass or more, or may be, for example, 11 parts mass or more, or 12 parts mass or more. Thus, using the soft intraocular lens material having a simpler structure, a soft intraocular lens in which both physical characteristics and a low glistening property are achieved at a high level can be realized.

Moreover, in a range in which the essence of the technology disclosed herein is not impaired, the (meth)acylate copolymer forming the soft intraocular lens material can include a polymer component corresponding to some other constituent unit than those described above.

Other examples of polymer components are, for example, an aromatic ring-containing acrylate, an alkoxy group-containing methacrylate, and a hydroxy group-containing methacrylate corresponding the aromatic ring-containing methacrylate (A), the alkoxy group-containing acrylate (B), and the hydroxy group-containing acrylate (C), respectively, that have been described above. However, because there is a possibility that these polymer components destabilize a balance of the above-described polymer components (A) to (C), it is preferable that a ratio of each of the polymer components to a corresponding one of the aromatic ring-containing methacrylate (A), the alkoxy group-containing acrylate (B), and the hydroxy group-containing acrylate (C) is 10 percent by mass or less and is more preferably 5 percent by mass or less, the is, for example, 3 percent by mass. A structure that does not include the aromatic ring-containing acrylate, the alkoxy group-containing methacrylate, and the hydroxy group-containing methacrylate is preferable.

Furthermore, still other examples of polymer components include, for example, a monomer represented by the formula: CH₂ = C(R¹)COOR². In this formula, R¹ is a hydrogen atom (H) or a methyl group (CH₃). R₂ in the formular is a linear, branched, or cyclic hydrocarbon group having 1 to 20 carbon atoms (C1-20). From a viewpoint of giving flexibility, R² preferably includes an alkyl (meth)acrylate that is an alkyl group having, for example, C1-12, typically C1-10, and preferably C2-8, that is, for example, C3-5. Specific examples of the alkyl (meth)acrylate include, for example, linear, branched, and cyclic alkyl (meth) acrylates, such as a methyl (meth)acrylate, an ethyl (meth)acrylate, a propyl (meth)acrylate, an n-butyl (meth)acrylate, a tert-butyl (meth)acrylate, an isobutyl (meth)acrylate, an n-pentyl (meth)acrylate, a tert-pentyl (meth)acrylate, a hexyl (meth)acrylate, a 2-methylbutyl (meth)acrylate, a heptyl (meth)acrylate, an octyl (meth)acrylate, a 2-ethylhexyl (meth)acrylate, a nonyl (meth)acrylate, a decyl (meth)acrylate, a dodecyl (meth)acrylate, a stearyl (meth)acrylate, a cyclopentyl (meth)acrylate, a cyclohexyl (meth)acrylate, or the like. One of these alkyl (meth)acrylates may be included alone, and two or more of the alkyl (meth)acrylates may be included in combination.

It is not preferable that an alkyl (meth)acrylate component is excessively contained because there is a probability that the balance of the above-described polymer components (A) to (C) is destabilized by excessively containing the alkyl (meth)acrylate component. Therefore, in a case where an alkyl (meth)acrylate component is contained, given that a total amount of polymer components is 100 parts mass, it is proper that the alkyl (meth)acrylate component is contained at a ratio of about 10 parts mass. The ratio of the alkyl (meth)acrylate component is typically about 5 parts mass or less, that is, for example, 3 parts mass or less and 1 parts mass or less, and, for example, a structure that substantially does not include the alkyl (meth)acrylate component is more preferable.

The (meta)acrylate copolymer can contain, as another polymer component, an ultraviolet-absorbing monomer component having an ultraviolet absorbing ability and a copolymerizability with the above-described polymer components (A) to (D). While a crystalline lens has a characteristic that the crystalline lens hardly transmits ultraviolet rays, the soft intraocular lens can transmit ultraviolet rays, and therefore, there is a risk of damaging a retina. Therefore, the ultraviolet absorbing ability can be imparted to the (meth)acrylate copolymer forming the soft intraocular lens 1 by incorporating as a sub-monomer component the ultraviolet-absorbing monomer component having the ultraviolet absorbing ability. Thus, the risk of damaging a retina can be suppressed, and also, a risk of developing an eye disease resulting from ultraviolet radiation, such as macular degeneration, corneitis, or the like, can be reduced. Note that the term "ultraviolet-absorbing monomer" refers to general monomers having an ultraviolet-absorbable functional group. Moreover, the term "ultraviolet-absorbable functional group" refers to functional atom groups having an absorption spectrum in an ultraviolet region. The ultraviolet-absorbing functional group can be a generic term for an alkyl residue, a carbonic acid residue, an alcohol residue, an amino residue, an acyl group, or the like of ultraviolet-absorbing compounds widely used as ultraviolet-absorbing agents. Examples of the ultraviolet-absorbing functional group widely range and include a carboxyl group or a hydroxyl group in an ultraviolet-absorbing compound, an atom group obtained by removing a hydrogen atom from an amino group or the like, an acyl group in an ultraviolet-absorbing compound, or the like. More specifically, as an ultraviolet-absorbing monomer component, a benzotriazole-based monomer component, a benzophenone-based monomer component, a salicylic acid-based monomer component, a cyanoacrylate-based monomer component can be preferably used.

It is proper that, given that a total amount of the polymer components is 100 parts mass, an ultraviolet-absorbing monomer component is contained at a ratio of about 0.1 to 1 parts mass. This is because, when a ratio of the ultraviolet-absorbing monomer component is excessively less than 0.1 percent by mass, the ultraviolet absorbing ability of the soft intraocular lens 1 is too low and there is a probability that an effective function cannot be achieved. Another reason for the foregoing is that, when the ratio of the ultraviolet-absorbing monomer component is excessively more than 1 percent by mass, there is a probability that the soft intraocular lens 1 is discolored due to a change in physical properties, a change with time, or the like. The ratio of the ultraviolet-absorbing monomer component is typically about 0.15 to 0.7 percent by mass, and is more preferably, for example, about 0.2 to 0.5 percent by mass.

The crystalline lens has a yellowish color, and therefore, has a characteristic of partially suppressing transmission of blue-color light that is opposite color. Therefore, the (meth)acrylate copolymer disclosed herein is preferably colored with a yellow color pigment, a red color pigment, or the like to adjust a color vision. As a coloring agent used for this purpose, a known azo-based compound, a known pyrazole-based compound, or the like having a copolymerizability with the (meth)acylate monomer component and being used as a coloring agent for this type of soft intraocular lens can be appropriately used. A blending ratio of these compounds can be appropriately determined based on a relationship with the above-described polymer components. For example, such a compound can be contained at a ratio in a range of about 0.001 to 0.1 parts mass to 100 parts mass of the polymer components.

Another compound used for forming the (meth) acrylate copolymer is, for example, a polymerization initiator. As the polymerization initiator, for example, a radical polymerization initiator formed of a benzoin ether or an amine can be preferably used. Specifically, for example, a polymerization initiator represented by a 2,2-azobisisobutyronitrile, an azobisdimethylvaleronitrile, a benzoin, a methyl orthobenzoyl benzoate, or the like can be used. A blending ratio of these compounds can be appropriately determined based on a relationship with the above-described polymer components. For example, such a compound can be contained at a ratio in a range of about 0.01 to 1 parts mass to 100 parts mass of the polymer components.

A method for obtaining the soft intraocular lens material is not particularly limited, and various polymerization methods known as methods for synthesizing a known (meth) acylate copolymer, such as a solution polymerization method, an emulsion polymerization method, a bulk polymerization method, a suspension polymerization method, a photopolymerization method, or the like, can be appropriately employed. Among these methods, for example, the solution polymerization method in which a mixed monomer composition obtained by appropriately blending the above-described polymer components is polymerized can be preferably used. A known production method, such as so-called cast molding method, lathe cutting method, or the like, can be appropriately employed for producing the soft intraocular lens 1. For example, in the cast molding method, specifically, a mold having a cavity corresponding to a shape of the soft intraocular lens 1 as a target may be prepared, a raw material composition (raw material monomer solution) may be supplied to the mold, and the raw material composition may be polymerized in the mold. For example, in the cutting method, specifically, a mixed monomer composition corresponding to a composition of the soft intraocular lens 1 as a target may be polymerized into a sheet shape (including a plate shape or the like) and a polymerized intraocular lens material in a sheet shape may be processed into a soft intraocular lens in a desired shape by cutting. The soft intraocular lens material in a sheet shape may be frozen and cutting may be performed thereon.

In the soft intraocular lens material disclosed herein, the occurrence of glistening has been remarkably reduced. For example, in a case where glistening is artificially caused to occur, the number of bright spots and an area thereof observed with a microscope have been reduced. For example, as a condition under which glistening is artificially caused to occur, the soft intraocular lens material is held at a temperature of 60 °C in a wet state, and thereafter, the temperature is changed to 25 °C.

The wet state can be understood as, for example, a state where the sort intraocular lens material is immersed in pure water. By immersing the soft intraocular lens material not in a physiological salt solution or the like but in pure water, glistening can be caused to occur in a short time. In the present specification, as pure water, super pure water whose electric resistivity at 25 °C is 15 MΩ·cm or more, or whose electric conductivity is 0.058 µS/cm) or less is preferably employed. A holding time in which the lens material is held in a high temperature environment at 60 °C can be, for example, about 2 hours as a standard. Thus, water can be sufficiently absorbed in the lens material in a state where a polymer structure of the lens material is relaxed. In other words, supersaturated water can be absorbed in the lens material.

Thereafter, temperature of the soft intraocular lens material is changed (cooled) to 25 °C. Thus, the polymer structure of the lens material is contracted (a water absorption rate is reduced) and moisture remaining in the polymer structure in a room temperature environment is condensed in a minute cavity. In order to stabilize the polymer structure after being cooled and a glistening occurrence state, the lens material after being cooled may be held at 25 °C for about 24 hours. Thus, glistening can be caused to acceleratively occur.

Note that, for "60 °C" and "25 °C" that are temperature conditions for the occurrence of glistening described above, fluctuations or errors of plus or minus 2 °C or so can be allowed.

At this time, in a known soft intraocular lens material, the entire lens material is clouded and the remarkable occurrence of bright spots throughout the lens material is found. For the known soft intraocular lens material, the number of bright spots of glistening found by observation with a microscope can be, for example, 1000/mm² or more per unit area. For example, an area of glistening found in the lens material having a diameter of about 6 mm can be more than 0.05 mm². On the other hand, in the soft intraocular lens material disclosed herein, in addition to a hydrophilic acrylate, a combination of the aromatic ring-containing methacrylate (A) and the alkoxy group-containing acrylate (B) preferably suppresses glistening. As a result, the number of bright spots of glistening per unit area found by observation with a microscope is suppressed to 400/mm² or less. Moreover, for example, an area pf glistening found in the lens material having a diameter of about 6 mm can be 0.05 mm² or less (for example, 0.045 mm² or less, and furthermore, 0.043 mm² or less).

In addition, the soft intraocular lens material disclosed herein has an optimal international rubber hardness for folding a lens, that is, 40 or more and 60 or less, and can be appropriately flexibly adjusted to an extent that is not too soft and not too hard. Such proper flexibility is realized by a proper combination of the aromatic ring-containing methacrylate (A) and the alkoxy group-containing acrylate (B). Thus, the soft intraocular lens material disclosed herein can be considered as a new soft intraocular lens material in which the occurrence of glistening is suppressed while proper flexibility is maintained, that is, both flexibility and glistening resistance are achieved.

Note that the soft intraocular lens 1 disclosed herein is constituted by a (meth)acrylate copolymer having the above-described structure, and therefore, similar to the known soft intraocular lens, preferably has foldable flexibility and elastic restoring force. Furthermore, in the soft intraocular lens 1 disclosed herein, the occurrence of glistening has been reduced. Based on the foregoing, the soft intraocular lens 1 is considered excellent in affinity in a biological environment. Moreover, even when the soft intraocular lens 1 is folded small, the soft intraocular lens 1 is developed to be restored to an original shape immediately after a folding force is released. Thus, the soft intraocular lens 1 disclosed herein can be preferably used particularly as a soft intraocular lens in a form in which the soft intraocular lens is bent small to be inserted to an eye by a dedicated insertion tool. In view of the foregoing, the technology disclosed herein can preferably provide this intraocular lens insertion system.

Although not specifically illustrated in the drawings, the intraocular lens insertion system includes the above-described soft intraocular lens 1 and an injector. The injector includes, for example, a syringe type injector body and a plunger. The injector body is configured such that a width of an internal space is reduced at a tip end side and the soft intraocular lens 1 is folded small in a width direction by passing through the internal space. The soft intraocular lens 1 is set inside the injector body in a flat state in advance. In inserting the soft intraocular lens 1 into a capsule or the like, a lubricant made of a viscoelastic material or the like is supplied to the injector body, and thereafter, the plunger is pressed into the injector body. Thus, the soft intraocular lens 1 is bent so as to be folded and is thus ejected out from an outlet at a tip end of the injector with the lubricant. Thus, the soft intraocular lens 1 can be easily inserted into the crystalline lens capsule of an eyeball. Note that the injector may be constituted by, for example, only a cartridge part of the injector body, that is, a part extending from a housing part that houses the soft intraocular lens 1 to the outlet from which the soft intraocular lens 1 is ejected. The cartridge part may be configured, for example, to be attachable and detachable with respect to the injector body.

### [Working Examples]

Several working examples related to the present invention will be described below, but there is no intention to limit the present invention to such specific examples.

As polymer components, raw material compositions for intraocular lens of Working Examples 1 to 19 (Working Examples 8 to 10 and 15 to 17 are not part of the invention) and Comparative Examples 1 to 13 were prepared by preparing the following polymerizable monomers with compositions illustrated in Table 1 and uniformly mixing the monomers with a polymerization initiator added thereto. Note that the raw material compositions of Working Examples 11 to 13 and Comparative Examples 11 to 13 contain two types of the alkoxy group-containing acrylate (B) but the raw material compositions of the other examples contain one type for each of the aromatic ring-containing methacrylate (A), the alkoxy group-containing acrylate (B), the hydroxy group-containing acrylate (C), and the cross-linkable (meth)acrylate (D). Each of these raw material components was poured into a mold including biconvex lens type pilotis and a molding flask (flat plate) and was polymerized at 60 °C for 12 hours and at 100 °C for 12 hours. Thus, first, a soft lens molded body of each of the examples for glistening evaluation was obtained. As for flat plate-shaped polymers, an intraocular lens material of each of the examples for international rubber hardness evaluation was obtained by cutting the each of the polymerized raw material components into a piece having a 1.5 cm square shape and a thickness of 1 mm.

As aromatic ring-containing monomers, the following 6 types were prepared.
BZMA: benzyl methacrylate
EGPEMA: ethylene glycol monophenyl ether methacrylate
EGPEA: ethylene glycol monophenyl ether acrylate
PEMA: phenylethyl methacrylate
PEA: phenylethyl acrylate

As aromatic ring-free monomers, the following 7 types were prepared.
MEA: 2-methoxyethyl acrylate
EEEA: 2-(2-ethoxyethoxy)ethyl acrylate
MEMA: 2-methoxyethyl methacrylate
EEMA: 2-ethoxyethyl methacrylate
EA: n-ethyl acrylate
BA: n-butyl acrylate
BMA: n- butyl methacrylate

As hydrophilic monomers, the following 3 types were prepared.
HBA: 4-hydroxybutyl acrylate
HEA: 2-hexyloxyethyl acrylate
HEMA: 2- hexyloxyethyl methacrylate

As cross-linkable monomers, the following 3 types were prepared.
BDDA: 1,4-butanediol diacrylate
NDDA: 1,9-nonanediol diacrylate
TMPTA: trimethylolpropane triacrylate

### [Measurement of IRHD]

For the intraocular lens material (in a 1.5 cm square shape) of each of the examples for evaluation, international rubber hardness (IRHD) was measured. As a durometer, GS-680sel (Type A durometer specified in JIS K6253:2012) manufactured by Teclock Co., Ltd was used and a plane hardness was measured in accordance with the M method (medium hardness micro size test) of IS048:2010 (JIS K6253-2). After the soft lens molded body of each of the examples was habituated for one hour or more in a test environment of temperature of 21 to 23 °C and humidity of 30 to 70% RH, the measurement of international rubber hardness was performed by measuring rubber hardness with test number: n =5. Arithmetic mean values were calculated from the obtained international rubber hardness, and results of the calculation are indicated in corresponding columns in Table 1. Note that a lower limit of IRHD is "30" and hardness less than IRHD 30 cannot be measured.

### [Evaluation of Glistening Suppression]

For the soft lens molded body of each of the examples for evaluation, glistening was caused to occur in an accelerating manner, and thereafter, the number of bright points of glistening and an area of the bright points were calculated by performing microscopic observation and image analysis to evaluate glistening characteristics. The soft lens molded body includes an optical part having a diameter of about 6 mm and a thickness of 0.6 mm and a pair of support parts located in a periphery of the optical part. As acceleration conditions for the occurrence of glistening, in a state where the soft lens molded body of each of the examples was immersed in super pure water, the soft lens molded body was held for 2 hours in a heating environment of 60 °C and subsequently was left to stand still at room temperature for 24 hours. By doing this, a water absorption rate of the lens molded body was rapidly reduced by giving a rapid temperature change from high temperature to low temperature to the lens molded body in a humid environment simulating an inside of a lens capsule, and thus, a situation where moisture was locally left to remain in a polymer structure was made. As the super pure water, super pure water (normally 18 MΩ·cm or more) whose electric resistivity was managed to be 16 MΩ·cm or more (°C) was used. Note that the room temperature was managed to be 25 ±2 °C.

For microscopic observation, by using a stereomicroscope SMZ1500 manufactured by Nikon Corporation and a digital camera DS-Vi1 manufactured by Nikon Corporation and controlling them using an image integration software NIS-Elements, a plane image (800 × 600 pixels) of each of the soft lens molded bodies for evaluation was obtained. In the image, an observation visual field was set such that bending parts of the support parts of the soft lend molded body were arranged to be diagonal and the optical part was arranged large in an entire area of the image. For image analysis, Image J developed by National Institutes of Health (NIH) in the USA.

Observation conditions of the stereomicroscope are as follow.
Mode setting: Manual exposure
Exposure time: 30 m/s
Gain: 1.00 ×
Contrast setting: Standard
Illumination: Dark field (light quantity maximum)
Light control dial: light quantity maximum
Magnification: 15 times (objective lens 1 ×, ocular lens 10 ×, zoom 1.5 ×)

Calculation of the number of bright points and the area of the bright points by image analysis was performed in accordance with the following procedures.

First, a photographed plane image of the soft lens molded body was taken into Image J, "8-bit" processing under a submenu Type (image type) under a menu Image was preformed thereon to convert the image to a bit grade scale image. Subsequently, binarization to convert pixels in a threshold range of the image to black and the other pixels to white was performed by adjusting an upper limit of a (threshold) level in Threshold to 255 and a lower limit thereof to 40 in a submenu Adjust (correction). Thus, the image of the soft lens molded body was binarized such that an outline and bright points were black and the other parts were white. Thereafter, an analysis region was created (Create Selection) by selecting a region (inside the outline) of the optical part of the binarized soft lens molded body image using an ellipsoidal region selection tool button in a (image) menu Image.

Next, using a submenu Analyze Particles under a menu Analyze, the number of objects (black points indicating bright points) and an area of the objects in the selected region were measured. A particle count and a total particle area that were calculated using this submenu were set as the number of bright points and a total area of the bright points, respectively.

Glistening suppression effects were evaluated from the number of bright points and the area that were obtained, based on indexes illustrated in Table 2 below, and results of the evaluation are indicated in a column of "Functionality evaluation" of Table 1. Note that, because the total particle area is represented in pixel unit, the total area was converted to that in mm² unit, based on a relationship of 1 mm = about 96 pixels obtained from the magnification of the image. For reference, calculation results of the total area are illustrated in Table 1.

### [Evaluation]

Based on the evaluation results for IRHD and glistening suppression described above, soft intraocular lens materials for which IRHD was 40 or more and 60 or less and a result of functionality evaluation of glistening was A were determined to be a non-defective (OK) having mechanical properties such as proper flexibility or the like and glistening suppression effects, soft intraocular lens materials for which IRHD was less than 40 and more than 60 and a result of functionality evaluation of glistening was B or C were determined to be a defective (NG), and the determination results were indicated in a column of "Evaluation" in Table 1.

**[Table 1]**

| No. | Aromatic ring-containing monomer A | | | Aromatic ring-free monomer B | | | Hydrophilic monomer | | Cross-linkable monomer | | Total | Hardness | Glistening | | Evaluation |
|---|---|---|---|---|---|---|---|---|---|---|---|---|---|---|---|
| | Monomer | Parts mass | BZA | Monomer | Parts mass | Parts mass | Monomer | Parts mass | Monomer | Parts mass | [Parts mass] | (IRHD) | Functionality evaluation | Area (mm²) | |
| Working Example 1 | BZMA | 40 | - | MEA | 46 | - | HBA | 10 | BDDA | 4 | 100 | 45 | A | 0.151 | OK |
| Working Example 2 | | 45 | - | | 41 | - | | 10 | | 4 | 100 | 44 | A | 0.129 | OK |
| Working Example 3 | | 49 | | | 35 | - | | 12 | | 4 | 100 | 45 | A | 0.253 | OK |
| Working Example4 | | 49 | | | 36 | - | | 10 | | 5 | 100 | 50 | A | 0.294 | OK |
| Working Example 5 | | 50 | - | | 36 | - | | 10 | | 4 | 100 | 46 | A | 0.329 | OK |
| Working Example 6 | | 51 | - | | 37 | - | | Θ | | 4 | 100 | 45 | A | 0.235 | OK |
| Working Example 7 | | 51 | - | | 36 | - | | 10 | | 3 | 100 | 40 | A | 0.213 | OK |
| Working Example 8 | | 51 | | | 32 | - | | 12 | | 5 | 100 | 52 | A | 0.175 | OK |
| Working Example 9 | | 52 | - | | 34 | - | | 10 | | 4 | 100 | 46 | A | 0.046 | OK |
| Working Example 10 | | 54 | | | 30 | - | | 12 | | 4 | 100 | 59 | A | 0.258 | OK |
| Comparative Example 1 | | 57 | | | **28** | - | | 10 | | 5 | 100 | 61 | A | 0.203 | **NG** |
| Working Example 11 | BZMA | 50 | - | MEA | 36 | - | HEA | 10 | BDDA | 4 | 100 | 47 | A | 0.147 | OK |
| Comparative Example 2 | | 50 | - | | 36 | - | **HEMA** | 10 | | 4 | 100 | 62 | C | **1.130** | **NG** |
| Working Example 12 | | 50 | - | | 36 | - | HBA | 10 | NDDA | 4 | 100 | 41 | A | 0.157 | OK |
| Working Example 13 | | 50 | - | | 36 | - | | 10 | TM PΓA | 4 | 100 | 49 | A | 0.353 | OK |
| Working Example 14 | BZMA | 50 | - | EEEA | 36 | - | HBA | 10 | BDDA | 4 | 100 | 45 | A | 0.154 | OK |
| Comparative Example 3 | | 50 | - | **MEMA** | **36** | - | | 10 | | 4 | 100 | 96 | A | 0.241 | NG |
| Comparative Example 4 | | 50 | - | **EEMA** | **36** | - | | 10 | | 4 | 100 | 94 | B | **0.436** | **NG** |
| Comparative Example 5 | | 50 | - | **BA** | **36** | - | | 10 | | 4 | 100 | 46 | B | **0.699** | **NG** |
| Comparative Example 6 | | 50 | - | **BMA** | **36** | - | | 10 | | 4 | 100 | 91 | A | 0.376 | **NG** |
| Working Example 15 | | 48 | | MEA + EEEA | 29 | 6 | | 12 | | 5 | 100 | 49 | A | 0.230 | OK |
| Working Example 16 | | 50 | | | 20 | 13 | | 12 | | 5 | 100 | 52 | A | 0.221 | OK |
| Working Example 17 | | 50 | | | 22 | 13 | | 10 | | 5 | 100 | 53 | A | 0.166 | OK |
| Working Example 18 | EGPEMA | 50 | - | MEA | 36 | - | HBA | 10 | BDDA | 4 | 100 | 46 | A | 0.397 | OK |
| Comparative Example 7 | **EGPEA** | 50 | - | | 36 | - | | 10 | | 4 | 100 | Unmeasurable | A | 0.288 | **NG** |
| Working Example 19 | PEMA | 50 | - | | 36 | - | | 10 | | 4 | 100 | 44 | A | 0.133 | **OK** |
| Comparative Example 8 | **PEA** | 50 | - | | 36 | - | | 10 | | 4 | 100 | Unmeasurable | A | 0.120 | **NG** |
| Comparative Example 9 | **EGPEA** | 50 | - | **EA** | - | **34** | **HEMA** | 13 | BDDA | 3 | 100 | **34** | C | **1.239** | **NG** |
| Comparative Example 10 | | 47 | - | | - | **31** | | 20 | | 2 | 100 | **31** | A | 0.157 | **NG** |
| Comparative Example 11 | | 50 | - | **EEMA + EA** | **17** | **17** | | 13 | | 3 | 100 | 38 | **B** | 0.524 | **NG** |
| Comparative Example 12 | | 50 | - | **EEMA** EEMA + EA | **34** | **-** | | 13 | | 3 | 100 | 40 | **B** | 0.480 | **NG** |
| Comparative Example 13 | | 49 | - | | **16** | **16** | | 16 | | 3 | 100 | 37 | A | 0.144 | **NG** |

Working Examples 8 and 9 (B = less than 35 parts mass), Working Example 10 (A = more than 52 parts mass, and B = less than 35 parts mass), and Working Examples 15, 16, and 17 (B = less than 35 parts mass) are not part of the invention.

**[Table 2]**

| Index | Number | Area |
|---|---|---|
| | [How many bright points] | [mm²] |
| A | 400 or less | 0.43 or less |
| B | More than 400 and 800 or less | More than 0.43 and 0.87 or less |
| C | More than 800 and 1200 or less | More than 0.87 |

As illustrated in Table 1, Working Examples 1 to 10 are examples in which BZMA that was an aromatic ring-containing methacrylate monomer, MEA that was an aromatic ring-free alkoxy group-containing acrylic monomer, HBA that was a hydrophilic acrylic monomer, and BDDA that was a cross-linkable acrylic monomer were combined and used while variously changing a blending ratio of these monomers. It was confirmed that, by employing the illustrated combinations of the monomers, soft intraocular lens materials in which both proper physical characteristics and glistening suppression effect have been achieved are obtained. It was also confirmed that, when, among main polymer components, either one of BZMA that is an aromatic ring-containing monomer and MEA that is an aromatic ring-free monomer is a main component (maximum component), excellent characteristics are realized. Furthermore, for example, as can be understood from comparison between Working Examples 6 to 8 or the like, it was confirmed that, in a system in which main polymer components are an aromatic ring-containing methacryl monomer and an aromatic ring-free alkoxy group-containing acrylic monomer, glistening is not always reduced as a content of a hydrophilic monomer increases. Accordingly, it is implied that a preferable combination of an aromatic ring-containing methacryl monomer and an aromatic ring-free alkoxy group-containing acrylic monomer contributes to reduction of glistening.

However, as indicated in Comparative Example 1, it was found that, given that a total content of the all polymer components is 100 parts mass, when, among the main polymer components, a content of MEA that is an acrylic monomer is lower than 30 parts mass, IRHD exceeds 60 and an obtained polymer is too hard in some cases. Among the main polymer components, a content of a softer acrylic component is preferably 30 parts mass or more.

In contrast, HBA used as a hydrophilic monomer was changed to HEA in Working Example 11 and to HEMA in Comparative Example 2. It was found that, in Working Example 11 in which HEA that was, as HBA was, an acrylic monomer component was used as a hydrophilic monomer, similar to Working Examples 1 to 10, a soft intraocular lens material in which both proper flexibility and glistening suppression effects have been achieved can be obtained. However, in Comparative Example 2 in which HEMA that was a methacryl monomer was used as a hydrophilic monomer, glistening abnormally occurred. A reason for this is considered that, because a hydrophilic methacryl monomer component existed on a surface of the polymer, a hydrophobic surface made of BZMA was not preferably formed and glistening was not controlled well. It was found that, in a system in which an aromatic ring-containing methacryl monomer and an aromatic ring-free alkoxy group-containing acrylic monomer are main polymer components, when a methacrylic monomer is mixed as a hydrophilic monomer, the occurrence of glistening cannot be preferably suppressed.

As a cross-linkable monomer, instead of BDDA having two functionalities and fewer carbons, NDDA having many carbons was used in Working Example 12 and TMPTA having three functionalities was used in Working Example 13. As for the cross-linkable monomer, even in a case where a functional group number, a chain length of a monomer, or the like was changed, a large change in flexibility and glistening suppression effects of the intraocular lens material was not observed. It was found that various types of the cross-linkable monomer may be used in combination with the above-described polymerizable monomers without any particular limitation on a type of the cross-linkable monomer.

For Working Example 14 and Comparative Examples 3 to 6, instead of MEA that was an aromatic ring-free alkoxy group-containing acrylic monomer, EEEA that was, as MEA was, an aromatic ring-free alkoxy group-containing acrylic monomer was used in Working Examples 14, MEMA and EEMA that were both methacrylates were used in Comparative Examples 3 and 4, and BA and BMA that were alkyl group-containing (meth)acrylate monomers were used in Comparative Examples 5 and 6. It was found that, in Working Example 14 using EEEA that is, as MEA is, an alkoxy acrylate monomer, similar to Working Examples 1 to 10, a soft intraocular lens material in which both proper flexibility and glistening suppression effects have been achieved can be obtained. However, in Comparative Examples 3 and 4 using, instead of the alkoxy acrylate monomer, MEMA and EEMA that are alkoxy methacrylate monomers, and furthermore, it was found that, in Comparative Example 6 using BMA that is an alkyl methacrylate monomer, IRHD largely increases and the flexibility of the soft intraocular lens material is lost. On the other hand, it was found that, in Comparative Example 5 using an alkyl acrylate monomer, although the flexibility is good but the occurrence of glistening cannot preferably be suppressed.

Note that, as indicated in Working Examples 15 to 17 (not part of the invention), it was confirmed that, even when two or more different monomer components, such as MEA, EEEA, or the like, are used in combination, similar effects can be achieved as long as the monomer components are aromatic ring-free alkoxy group-containing acrylic monomers.

Based on the foregoing, it was confirmed that, whether an acrylate or a methacrylate is selected as a monomer component is has a large effect on the physical characteristics and the glistening characteristics of a polymer to be formed.

Subsequently, for Working Examples 18 and 19 and Comparative Examples 7 and 8, instead of BZMA that was an aromatic ring-containing methacrylate, EGPEMA and EGPEA were used in Working Example 18 and Comparative Example 7, and PEMA and PEA were used in Working Example 19 and Comparative Example 8, respectively. It was found that, in Working Examples 18 and 19 using EGPEMA or PEMA that is an aromatic ring-containing methacrylate, a soft intraocular lens material in which both excellent physical characteristics and glistening suppression effects have been achieved can be obtained. However, in Comparative Examples 7 and 8 using EGPEA and PEA that are the above-described acrylates, obtained polymers were too soft, and IRHD could not be measured. It was found that, unless an aromatic ring-containing monomer that is combined with an aromatic ring-free alkoxy group-containing acrylic monomer is a methacryl monomer, it is difficult to handle a soft intraocular lens material.

As described above, each monomer component is an acrylate monomer or a methacrylate monomer, and thus, characteristics of an obtained soft intraocular lens material are largely changed. Herein, in Comparative Examples 9 to 13, a combination of an acrylate and a methacrylate among an aromatic ring-containing monomer, an aromatic ring-free monomer, a hydrophilic monomer, and a cross-linkable monomer was variously changed. As a result, it was found that, for example, in a case where EGPEA is used as the aromatic ring-containing monomer, as indicated in Comparative Examples 9 to 11, and 13, when any one of EA, EEMA, and a combination of EA and EEMA is used as the aromatic ring-free monomer, IRHD tends to be low, that is, less than 40, and there is a probability that, although the flexibility of a soft intraocular lens is high, it takes time to restore an original shape of the folded lend in a lens capsule and the original shape is not completely restored. At the same time, it is concerned that the lens is easily damaged when the lens is injected from an injector. Note that, for the soft intraocular lens of Comparative Example 12, it was found that IRHD is proper, that is, 40, but glistening tends to occur, and both physical characteristics and low glistening characteristics cannot be achieved. Based on the foregoing, it was found that, when a combination of monomers that are used is changed even slightly, a balance of IRHD and/or glistening suppression effects is lost, and a high quality soft intraocular lens material cannot be provided. Therefore, it was found that, in the technology disclosed herein, it is important to form a soft intraocular material by combining an aromatic ring-containing methacryl monomer, an aromatic ring-free alkoxy group-containing acrylic monomer, and a hydrophilic acrylic monomer.

Specific examples of the present invention have been described in detail above, but these are merely examples and do not limit the scope of the claims.

### Reference Signs List

- 1: Intraocular lens
- 10: Optical part
- 20: Support parts
- 20: Bending part

## Claims

1. An intraocular lens material obtained by polymerizing polymerizable polymer components, wherein
the polymer components include
an aromatic ring-containing methacrylate (A),
an alkoxy group-containing acrylate (B),
a hydroxy group-containing acrylate (C), and
a cross-linkable (meth)acrylate (D), and,
given that a total content of the polymer components is 100 parts mass, a ratio of the aromatic ring-containing methacrylate (A) is 40 parts mass or more and 52 parts mass or less,
given that a total content of the polymer components is 100 parts mass, a ratio of the alkoxy group-containing acrylate (B) is 35 parts mass or more and 46 parts mass or less,
given that a total content of the polymer components is 100 parts mass, a ratio of the hydroxy group-containing acrylate (C) is 8 parts mass or more and 12 parts mass or less,
given that a total content of the polymer components is 100 parts mass, a ratio of the cross-linkable (meth)acrylate (D) is 1 part mass or more and 10 parts mass or less, wherein
the aromatic ring-containing methacrylate (A) contains a methacrylate represented by a general formula (1): 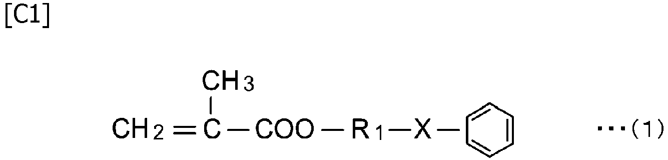
where R₁ is a linear or branched alkylene group having 1 to 8 carbon atoms and X indicates that an element is absent or is an oxygen atom,
the alkoxy group-containing acrylate (B) contains an acrylate represented by a general formula (2): 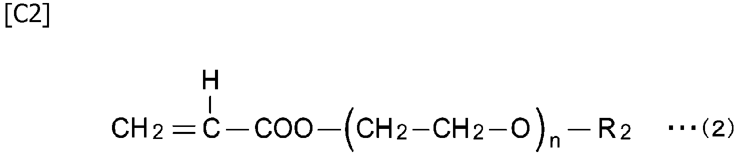
where R₂ is a methyl group or an ethyl group and n is an integer of 1 to 4,
the hydroxy group-containing acrylate (C) contains an acrylate represented by a general formula (3): 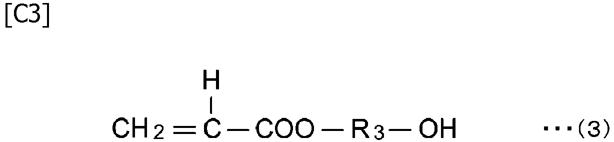
where R₃ is a linear or branched alkylene group having 1 to 8 carbon atoms, and
the cross-linkable (meth)acrylate (D) is a bifunctional (meth)acrylate or a trifunctional (meth)acrylate.

2. The intraocular lens material according to claim 1, wherein
given that a total content of the polymer components is 100 parts mass, a total among of the aromatic ring-containing methacrylate (A) and the alkoxy group-containing acrylate (B) is 75 parts mass or more and 90 parts mass or less.

3. The intraocular lens material according to claim 1 or 2, wherein
the polymer components include a monomer having a yellow coloring ability and a copolymerizability with the (meth)acylate monomer component, wherein the monomer is an azo-based compound or a pyrazole-based compound.

4. A soft intraocular lens produced using the intraocular lens material of any one of claims 1 to 3.

## Patentansprüche

1. Intraokularlinsenmaterial, erhalten durch Polymerisieren polymerisierbarer Polymerkomponenten, wobei die Polymerkomponenten
ein einen aromatischen Ring enthaltendes Methacrylat (A),
ein eine Alkoxygruppe enthaltendes Acrylat (B),
ein eine Hydroxygruppe enthaltendes Acrylat (C) und
ein vernetzbares (Meth)acrylat (D) umfassen, und
bei einem Gesamtgehalt der Polymerkomponenten von 100 Masseteilen ein Anteil des den aromatischen Ring enthaltenden Methacrylats (A) 40 Masseteile oder mehr und 52 Masseteile oder weniger,
bei einem Gesamtgehalt der Polymerkomponenten von 100 Masseteilen ein Anteil des die Alkoxygruppe enthaltenden Acrylats (B) 35 Masseteile oder mehr und 46 Masseteile oder weniger,
bei einem Gesamtgehalt der Polymerkomponenten von 100 Masseteilen ein Anteil des die Hydroxygruppe enthaltenden Acrylats (C) von 8 Masseteilen oder mehr und 12 Masseteilen oder weniger,
bei einem Gesamtgehalt der Polymerkomponenten von 100 Masseteilen ein Anteil des vernetzbaren (Meth)acrylats (D) 1 Masseteil oder mehr und 10 Masseteile oder weniger beträgt, wobei
das den aromatischen Ring enthaltende Methacrylat (A) ein Methacrylat enthält, das durch die allgemeine Formel (1) dargestellt wird: 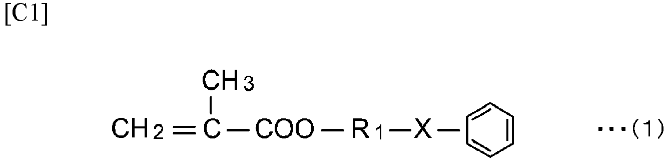
wobei Ri eine lineare oder verzweigte Alkylengruppe mit 1 bis 8 Kohlenstoffatomen ist und X anzeigt, dass ein Element fehlt oder ein Sauerstoffatom ist,
das die Alkoxygruppe enthaltende Acrylat (B) ein Acrylat enthält, das durch die allgemeine Formel (2) dargestellt wird: 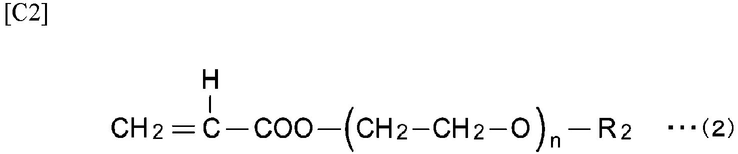
worin R₂ eine Methylgruppe oder eine Ethylgruppe ist und n eine ganze Zahl von 1 bis 4 ist,
das Hydroxygruppen enthaltende Acrylat (C) ein Acrylat enthält, das durch die allgemeine Formel (3) dargestellt wird: 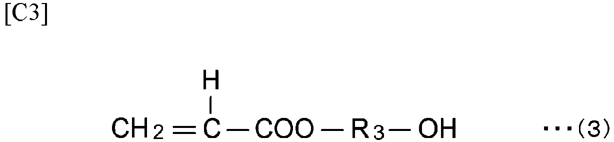
worin R₃ eine lineare oder verzweigte Alkylengruppe mit 1 bis 8 Kohlenstoffatomen ist, und
das vernetzbare (Meth)acrylat (D) ein bifunktionelles (Meth)acrylat oder ein trifunktionelles (Meth)acrylat ist.

2. Intraokularlinsenmaterial nach Anspruch 1, wobei
bei einem Gesamtgehalt der Polymerkomponenten von 100 Masseteilen ein Gesamtgehalt des den aromatischen Ring enthaltenden Methacrylats (A) und des die Alkoxygruppe enthaltenden Acrylats (B) 75 Masseteile oder mehr und 90 Masseteile oder weniger beträgt.

3. Intraokularlinsenmaterial nach Anspruch 1 oder 2, wobei
die Polymerkomponenten ein Monomer mit der Fähigkeit zur Gelbfärbung und einer Copolymerisierbarkeit mit der (Meth)acrylat-Monomerkomponente umfassen, wobei das Monomer eine Verbindung auf Azobasis oder eine Verbindung auf Pyrazolbasis ist.

4. Weiche Intraokularlinse, hergestellt unter Verwendung des Intraokularlinsenmaterials nach einem der Ansprüche 1 bis 3.

## Revendications

1. Matériau de lentille intraoculaire obtenu par polymérisation de composants polymères polymérisables, dans lequel
les composants polymères comprennent
un méthacrylate contenant un cycle aromatique (A),
un acrylate contenant un groupe alkoxy (B),
un acrylate contenant un groupe hydroxy (C), et
un (méth)acrylate réticulable (D), et
étant donné que la teneur totale des composants du polymère est de 100 parties en masse, le rapport du méthacrylate contenant un cycle aromatique (A) est de 40 parties en masse ou plus et de 52 parties en masse ou moins,
étant donné que la teneur totale des composants du polymère est de 100 parties en masse, le rapport de l'acrylate contenant un groupe alkoxy (B) est supérieur ou égal à 35 parties en masse et inférieur ou égal à 46 parties en masse,
la teneur totale des composants du polymère étant de 100 parties en masse, le rapport de l'acrylate contenant un groupe hydroxy (C) est supérieur ou égal à 8 parties en masse et inférieur ou égal à 12 parties en masse,
étant donné que la teneur totale des composants du polymère est de 100 parties en masse, le rapport du (méth)acrylate réticulable (D) est de 1 partie en masse ou plus et de 10 parties en masse ou moins, dans lequel
le méthacrylate contenant un cycle aromatique (A) contient un méthacrylate représenté par la formule générale (1) : 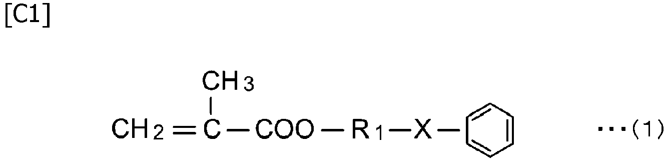
où R₁ est un groupe alkylène linéaire ou ramifié ayant de 1 à 8 atomes de carbone et X indique qu'un élément est absent ou est un atome d'oxygène,
l'acrylate contenant un groupe alkoxy (B) contient un acrylate
représenté par la formule générale (2) : 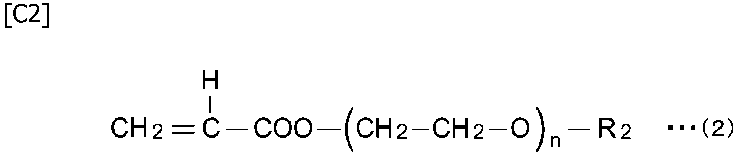
où R₂ est un groupe méthyle ou un groupe éthyle et n est un nombre entier de 1 à 4,
l'acrylate contenant un groupe hydroxy (C) contient un acrylate représenté par la formule générale (3) : 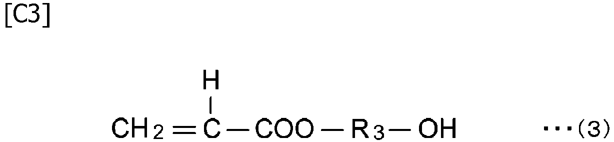
où R₃ est un groupe alkylène linéaire ou ramifié ayant de 1 à 8 atomes de carbone, et
le (méth)acrylate réticulable (D) est un (méth)acrylate bifonctionnel ou un (méth)acrylate trifonctionnel.

2. Le matériau de lentille intraoculaire selon la revendication 1, dans lequel
étant donné que la teneur totale des composants polymères est de 100 parties en masse, la teneur totale du méthacrylate contenant un cycle aromatique (A) et de l'acrylate contenant un groupe alkoxy (B) est de 75 parties en masse ou plus et de 90 parties en masse ou moins.

3. Le matériau de lentille intraoculaire selon la revendication 1 ou 2, dans lequel
les composants polymères comprennent un monomère ayant une capacité de coloration jaune et une capacité de copolymérisation avec le composant monomère (méth)acylate, dans lequel le monomère est un composé à base d'azoïque ou un composé à base de pyrazole.

4. Lentille intraoculaire souple fabriquée à l'aide du matériau de lentille intraoculaire de l'une des revendications 1 à 3.
